# EUROPEAN PATENT APPLICATION

(11) **EP 3 199 099 A1**
(43) Date of publication of application: **02.08.2017**
(21) Application number: 16191206.8
(22) Date of filing: 28.09.2016
(51) Int. Cl.: A61B 5/00, A61B 5/04, A61B 5/0408

(54) **PHYSIOLOGICAL MONITORING DEVICE AND SYSTEM USING THE SAME**

(30) Priority: 01.02.2016 TW 105103092
(71) Applicant: Rooti Labs Limited, 1-1112 Grand Cayman (KY)
(72) Inventor: CHANG, Ming-Shiung, New Taipei (TW)
(74) Representative: Huang, Chongguang

(57) **Abstract**

A physiological monitoring device includes a plurality of conducting plates, a printed circuit board (PCB), a paster, and an information collector. The conducting plates are configured to be adhesively attached to a detecting portion of a living subject, such as a patient, to detect physiological characteristics. The PCB is configured to be electrically coupled to the plurality of conducting plates. The conducting plates are fastened on terminals of the PCB. The paster plate is arranged on the PCB, and electrically coupled to the PCB. The information collector is detachably coupled to the paster, and is electrically coupled to the PCB through the paster. The physiological monitoring device can be conveniently used whenever and wherever possible. A system applying the physiological monitoring device is also disclosed.

## Description

### CROSS-REFERENCE TO RELATED APPLICATIONS

This application claims priority to Taiwanese Patent Application No. 105103092 filed on February 1, 2016 in the Taiwan Intellectual Property Office, the contents of which are incorporated by reference herein.

### FIELD

The subject matter herein generally relates to physiological monitoring devices and systems using the same.

### BACKGROUND

Sensor devices have been used in hospital or community nursing service to detect a variety of physiological characteristics of the living subjects, such as the patients. Conventional sensor devices can detect some portions of living subjects via a plurality of wires and interfaces.

### SUMMARY

A physiological monitoring device includes a plurality of conducting plates, a printed circuit board (PCB), a paster, and an information collector. The conducting plates are configured to paste on a detecting portion to detect physiological characteristics. The PCB is configured to electrically coupled to the plurality of conducting plates. The conducting plates are fastened on terminals of the PCB. The paster plate is arranged on the PCB, and electrically coupled to the PCB. The information collector is detachably coupled to the paster, and is electrically coupled to the PCB through the paster. The physiological monitoring device can be conveniently used whenever and wherever possible.

### BRIEF DESCRIPTION OF THE DRAWINGS

Implementations of the present disclosure will now be described, by way of example only, with reference to the attached figures.
FIG. 1 is a schematic view of a physiological monitoring system according to one embodiment, wherein the physiological monitoring system includes a physiological monitoring device.
FIG. 2 is an exploded view of the physiological monitoring device shown in FIG. 1, wherein the physiological monitoring device includes an information collector.
FIG. 3 is an exploded view of the information collector of the physiological monitoring device shown in FIG. 2 from one angle.
FIG. 4 is an exploded view of the information collector shown in FIG. 3 from another angle.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures and components have not been described in detail so as not to obscure the related relevant feature being described. Also, the description is not to be considered as limiting the scope of the embodiments described herein. The drawings are not necessarily to scale and the proportions of certain parts may be exaggerated to better illustrate details and features of the present disclosure.

Several definitions that apply throughout this disclosure will now be presented.

The term "comprising" when utilized, means "including, but not necessarily limited to"; it specifically indicates open-ended inclusion or membership in the so-described combination, group, series and the like.

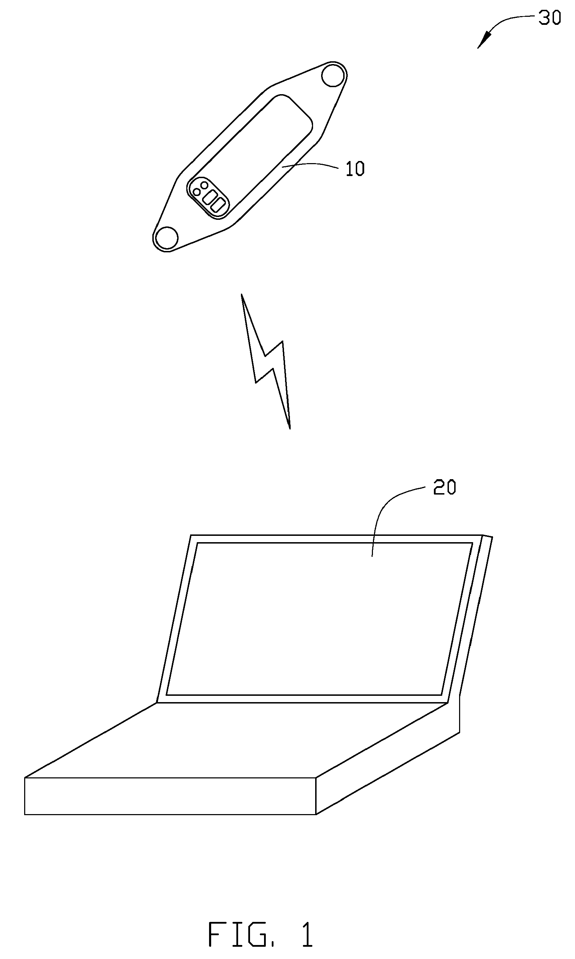

FIG. 1 illustrates schematic view of one embodiment of a physiological monitoring system 30 including a physiological monitoring device 10 and a terminal device 20. The physiological monitoring device 10 is coupled to portion of a living subject to detect a plurality of physiological characteristics, and is coupled to the terminal device 20 via wireless connections or wired connections to transmit a variety of vital signals to the terminal device 20. In at least one embodiment, the terminal device 20 can be a medical treatment device, a mobile phone, a computer, a server or a cloud host.

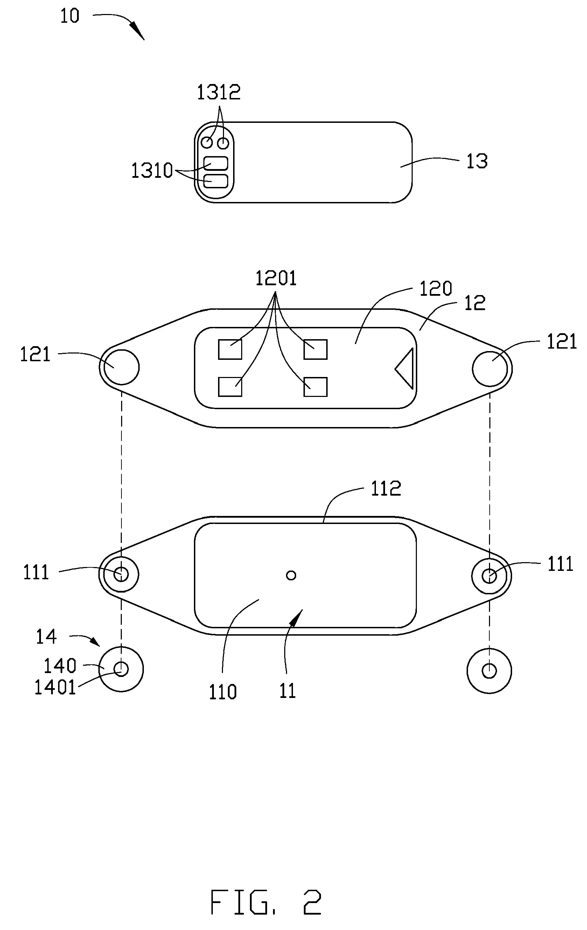

FIG. 2 illustrates an exploded view of the physiological monitoring device 10. The physiological monitoring device 10 includes a printed circuit board (PCB) 11, a paster 12, an information collector 13, and a plurality of conducting plates 14. The paster 12 and the information collector 13 are arranged on the PCB 11 sequentially. The PCB 11 is pasted on a detecting portion of the living subject via the conducting plates 14, to detect the plurality of physiological characteristics. In at least one embodiment, the PCB 11 is a flexible printed circuit board.

Each of the conducting plates 14 includes a first side surface (not labeled) and a second side surface 140 opposite to the first side surface. Both of the first side surface and the second side surface are made of waterproof material, such as waterproof tape. In at least one embodiment, number of the conducting plates 14 is two. Each second side surface 140 includes a signal terminal 1401 with a corresponding monitoring electrode, to sense the plurality of physiological characteristics of the living subject.

The PCB 11 includes a main body 110 and two first connection portions 111 set on two terminal of the main body 110. The main body 110 includes at least one waterproof tape 112 set on surface of the main body 110. The PCB 11 can be pasted on the detecting portion of the living subject through the waterproof tape 112. Each one of the two first connection portions 111 includes a first side wall and a second side wall. The first side wall of each first connection portion 111 is fastened to one corresponding signal terminal 1401, to monitor the detecting portion of the living subject through the corresponding signal terminal 1401.

A shape of the paster 12 is the same as that of the PCB 11. The paster 12 includes an install area 120 and two second connection portions 121 being set on two terminals of the install area 120 respectively. In at least one embodiment, the second side wall of each first connection portion 111 is coupled to one corresponding second connection portion 121. Both of the first connection portions 111 and the second connection portions 121 are made of metal fasteners, and are electrically coupled to each other in manner of fastening of metal fasteners. The install area 120 of the paster 12 sets a plurality of conductive terminals 1201. In at least one embodiment, number of the conductive terminals 1201 is four. Each one of the conductive terminals 1201 is a conductive tape, and is electrically coupled to the second connection portions 121, to monitor the detecting portion of the living subject through the first connection portions 111, the second connection portions 121, and the conducting plate 14. The information collector 13 is detachably coupled to the install area 120 of the paster 12 through the conductive terminals 1201.

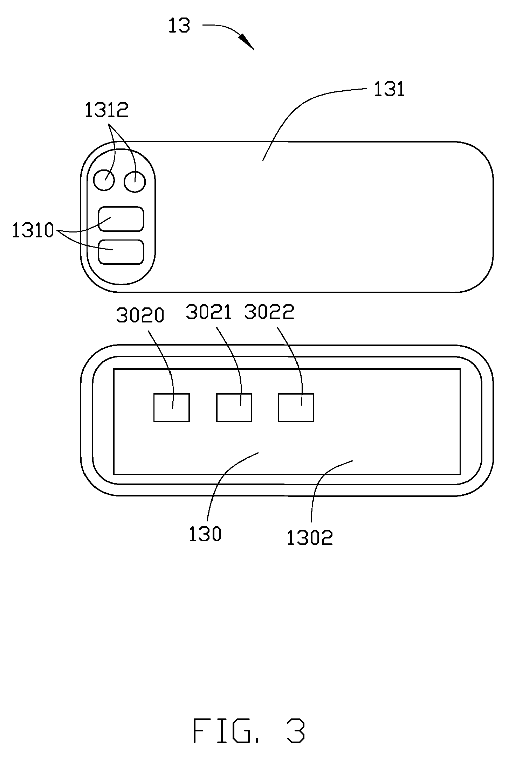

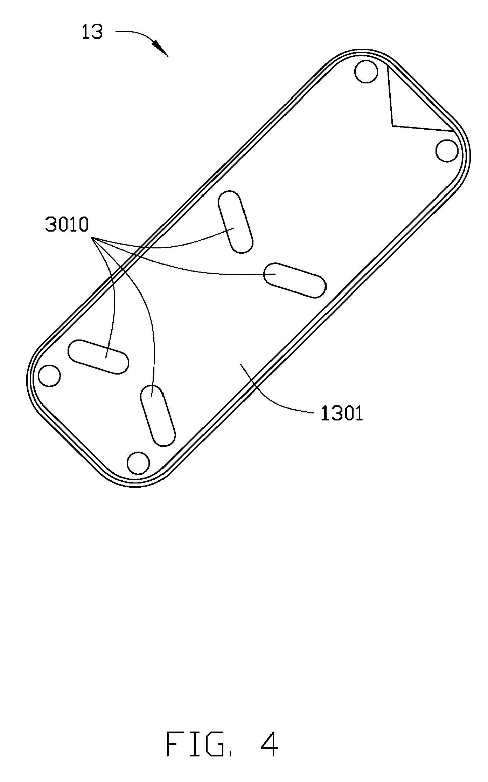

Referring FIGS. 3 and 4, the information collector 13 includes a first cover 130 and a second cover 131 coupled to the first cover 130. The first cover 130 is a circuit board, and includes an outer surface 1301 and an inner surface 1302 opposite to the outer surface 1301. The outer surface 1301 includes four electrodes 3010 coupled to the four conductive terminals 1201 of the install area 120 correspondingly. The inner surface 1302 of the first cover 130 includes a power module 3020, a processing circuit 3021, and a wireless module 3022. The power module 3020 is configured to supply power to the processing circuit 3021 and the wireless module 3022. The processing circuit 3021 is electrically coupled to the four electrodes 3010 and the wireless module 3022, to receive signals of the physiological characteristics of the living subject via the electrodes 3010 coupled to the conductive terminals 1201. The processing circuit 3021 controls the signals of the physiological characteristics being amplified, filtered, coded, decoded, and then transmitted to the terminal device 20 through the wireless module 3022. The second cover 131 includes a plurality of function keys 1310, a plurality of indicator lights 1312, and a plurality of USB interface (not labeled). In at least one embodiment, the function keys 1310 include, but not limited, a power switch key and a wireless transmission key. The power switch key is electrically coupled to the processing circuit 3021 and the power module 3020, to enabled the processing circuit 3021 and the power module 3020. Number of the indicator lights 1312 is two, which are configured to indicate opening status of the power switch key and the wireless transmission key. The USB interface is electrically coupled to the power module 3020 to supply power to the power module 3020, through a data transmission line coupled to an external power supply.

In use, when the two conducting plates 14 are coupled to the two first connection portions 111 correspondingly, the two first connection portions 111 of the PCB 11 are fastened on the two second connection portions 121 correspondingly, the information collector 13 is pasted on the install area 120 of the paster 12. Plastic films set on the waterproof tapes of the PCB 11 and the conducting plate 14 can be taken down by user, and the physiological monitoring device 10 is pasted on the detecting portion of the living subject. The power module 3020, the processing circuit 3021, and the wireless module 3022 can be enabled to operate by pressing the power switch key and the wireless transmission key.

When the power switch key is enabled, the power module 3020 supplies power to the processing circuit 3021, and the processing circuit 3021 outputs a control signal to the monitoring electrodes of the two signal terminals 1401 through the four electrodes 3010, the plurality of conductive terminals 1201, two first connection portions 111, and two second connection portions 121, to enable the monitoring electrodes to detect physiological characteristics of the living subject. The two signal terminals 1401 detect the signals of the physiological characteristics of the living subject, and transmit the detected signals to the processing circuit 3021 of the information collector 13 through the two first connection portions 111, two second connection portions 121, the plurality of conductive terminals 1201, and the four electrodes 3010. The processing circuit 3021 controls the signals of the physiological characteristics being amplified, filtered, coded, decoded, and then stored in a storage of the processing circuit 3021. When the wireless transmission key is enabled, the signals of the physiological characteristics stored in the storage can be transmitted to the terminal device 20 through the wireless module 3022.

When both of the power switch key and the wireless transmission key are enabled simultaneously, the signals the physiological characteristics of the living subject can be transmitted to the terminal device 20 through the wireless module 3022, after being amplified, filtered, coded, and decoded by the processing circuit 3021.

Therefore, user can selectively enable the power switch key and the wireless transmission key simultaneously or non-simultaneously, according to an operational environment, such as in showering.

When the physiological monitoring device 10 does not operate, the information collector 13 can be taken down from the paster 12, and be coupled to the external power supply through the USB interface and the data transmission line, to power the power module 3020 of the information collector 13.

Therefore, the physiological monitoring device 10 can be conveniently used whenever and wherever possible. Furthermore, either of the PCB 11, the paster 12, the information collector 13, and the two conducting plates 14 is independent and waterproof, which can make the physiological monitoring device 10 can be used in showering, and any one component of the physiological monitoring device 10 can be changed and repaired in targeted to reduce using cost of the physiological monitoring device 10.

The embodiments shown and described above are only examples. Therefore, many such details are neither shown nor described. Even though numerous characteristics and advantages of the present technology have been set forth in the foregoing description, together with details of the structure and function of the present disclosure, the disclosure is illustrative only, and changes may be made in the details, especially in matters of shape, size and arrangement of the parts within the principles of the present disclosure up to, and including the full extent established by the broad general meaning of the terms used in the claims. It will therefore be appreciated that the embodiments described above may be modified within the scope of the claims.

## Claims

1. A physiological monitoring device comprising:
a plurality of conducting plates configured to be pasted on a detecting portion, which is configured to detect physiological characteristics;
a printed circuit board (PCB) comprising a plurality of first connection portions, one side of the plurality of first connection portions coupled to corresponding ones of the plurality of conducting plates;
a paster comprising an install area and a plurality of second connection portions correspondingly coupled to another side of the plurality of first connection portions, the install area comprising a plurality of conductive terminals; and
an information collector detachably coupled to the install area of the paster and comprising a plurality of electrodes, wherein the plurality of electrodes are correspondingly coupled to the plurality of conductive terminals, are configured to be electrically coupled to the plurality of conducting plates through, in sequence, the conductive terminals, the second connection portions, and the first connection portions.

2. The physiological monitoring device according to claim 1, wherein the plurality of conductive terminals are conductive tape.

3. The physiological monitoring device according to claim 2, wherein the first connection portions are disposed at two terminals of the PCB, the second connection portions are disposed at two terminals of the paster, the first connection portions, the conductive terminals, and the second connection portions are fastened together correspondingly.

4. The physiological monitoring device according to claim 3, wherein each of the conducting plates comprises a signal terminal with a monitoring electrode to detect physiological characteristics, each signal terminal is coupled to one first connection portion correspondingly.

5. The physiological monitoring device according to claim 4, wherein both surfaces of the PCB and the paster comprise waterproof material.

6. The physiological monitoring device according to claim 5, wherein the waterproof material is waterproof tape.

7. The physiological monitoring device according to claim 1, wherein information collector comprises a wireless module, the information collector receives signals of the physiological characteristics through the plurality of electrodes and transmits the signals of the physiological characteristics to a terminal device through the wireless module.

8. The physiological monitoring device according to claim 7, wherein information collector further comprises a power module and a processing circuit, the power module is configured to supply power to the processing circuit and the wireless module, the processing circuit is electrically coupled to the plurality of electrodes and the wireless module, to receive signals of the physiological characteristics through the plurality of electrodes and transmit the signals of the physiological characteristics to the terminal device through the wireless module.

9. A physiological monitoring device comprising:
a plurality of conducting plates configured to paste on a detecting portion to detect physiological characteristics;
a printed circuit board (PCB) configured to electrically coupled to the plurality of conducting plates, the conducting plates fastened on the PCB;
a paster arranged on the PCB, and electrically coupled to the PCB; and
an information collector detachably coupled to the paster, and electrically coupled to the PCB through the paster.

10. The physiological monitoring device according to claim 9, wherein the paster comprises a plurality of conductive terminals, the information collector comprises a plurality of electrodes, the plurality of conductive terminals are coupled to the plurality of electrodes correspondingly.

11. The physiological monitoring device according to claim 10, wherein the conductive terminals are conductive tape.

12. The physiological monitoring device according to claim 9, wherein the PCB comprises two terminals, the conducting plates are fastened on the two terminals of the PCB.

13. The physiological monitoring device according to claim 12, wherein the paster has a same shape as that of the PCB and comprises two terminals, the two terminals of the paster are fastened on the two terminals of the PCB.

14. A physiological monitoring system comprising:
a physiological monitoring device comprising:
a plurality of conducting plates configured to be pasted on a detecting portion, which is configured to detect physiological characteristics;
a printed circuit board (PCB) comprising a plurality of first connection portions, one side of the plurality of first connection portions coupled to corresponding ones of the plurality of conducting plates;
a paster comprising an install area and a plurality of second connection portions correspondingly coupled to another side of the plurality of first connection portions, the install area comprising a plurality of conductive terminals;
an information collector detachably coupled to the install area of the paster, and comprising a plurality of electrodes, wherein the plurality of electrodes of the information collector are correspondingly coupled to the plurality of conductive terminals, are configured to be electrically coupled to the plurality of conducting plates through, in sequence, the conductive terminals, the second connection portions, and the first connection portions; and
a terminal device receiving signals of the physiological characteristics from the physiological monitoring device.

15. The physiological monitoring system according to claim 14, wherein terminal device receives signals of the physiological characteristics from the physiological monitoring device through connection of wireless.
